(19) Europäisches Patentamt
European Patent Office
Office européen des brevets

(11) Veröffentlichungsnummer: **0 009 559**
**B1**

(12) **EUROPÄISCHE PATENTSCHRIFT**

(45) Veröffentlichungstag der Patentschrift: **25.08.82**

(51) Int. Cl.³: **A 61 K 9/00**

(21) Anmeldenummer: **79102682.6**

(22) Anmeldetag: **27.07.79**

(54) **Stabile flüssige Arzneimittelformulierung, ihre Herstellung und Verwendung.**

(30) Priorität: **01.08.78 CH 8217/78**

(43) Veröffentlichungstag der Anmeldung:
**16.04.80 Patentblatt 80/8**

(45) Bekanntmachung des Hinweises auf die Patenterteilung:
**25.08.82 Patentblatt 82/34**

(84) Benannte Vertragsstaaten:
**BE CH DE FR GB IT NL**

(56) Entgegenhaltungen:
**DE-A-2 615 140**
**DE-A-2 627 706**
**DE-A-2 631 779**
**DE-A-2 631 780**
**DE-A-2 712 198**
**DE-B-1 028 292**
**US-A-3 728 452**
**US-A-4 018 889**
**ORGANISCH-CHEMISCHE ARZNEIMITTEL UND IHRE SYNONYMA«, Martin Negwer, Band I, Adademie Verlag, Berlin, DE, 1971, Seiten 174–175**

(73) Patentinhaber: **CIBA-GEIGY AG, Patentabteilung Postfach, CH-4002 Basel (CH)**

(72) Erfinder: **Lukas, Gerhard, Dr., In den Wegscheiden 16, CH-4132 Muttenz (CH)**
Erfinder: **Atasoy, Kaya, Dr., Loogstrasse 32, CH-4142 Münchenstein (CH)**

(74) Vertreter: **Zumstein, Fritz sen., Dr. et al, Bräuhausstrasse 4, D-8000 München 2 (DE)**

**EP 0 009 559 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Stabile flüssige Arzneimittelformulierung, ihre Herstellung und Verwendung

Die vorliegende Erfindung betrifft eine flüssige Formulierung enthaltend eine Wirkstoffkombination aus 2,6-Diamino-5-(3′,4′,5′-trimethoxybenzyl)-pyrimidin (Trimethoprim) und einem Sulfonamid zur Bekämpfung von bakteriellen und protozoären Krankheiten bei Tieren mittels Trinkwasserapplikation. Außer den genannten Wirkstoffen enthält die Formulierung ferner ein organisches Lösungsmittel, einen Lösungsvermittler und ein Tensid.

Die Wirksamkeit von Sulfonamid-Trimethoprim-Kompositionen in der Bekämpfung von Infektionskrankheiten bei Mensch und Tier ist gut bekannt. Die besondere Bedeutung der Trimethoprim-Komponente beruht auf der Potenzierung der antibakteriellen bzw. protozoären Wirksamkeit der Sulfonamide.

Die Verabreichung solcher Wirkstoffkombinationen an Tiere stößt indessen auf mancherlei Schwierigkeiten. So ist bei der Applikation in fester Form durch Zugabe zur Alimentation in den überwiegenden Fällen eine erfolgversprechende Therapie nicht möglich, da erkrankte Tiere im allgemeinen die Futteraufnahme stark reduzieren, wenn nicht gar gänzlich verweigern. Die Wasseraufnahme erleidet dagegen normalerweise keine Einschränkung, so daß sich durch Medikierung des Trinkwassers ein Weg für die enterale Einschleusung von Wirkstoffen in den Tierorganismus anbietet.

Eine weitere Möglichkeit ist die parenterale Wirkstoffapplikation mittels Injektion, die jedoch bei prophylaktischer Anwendung auf zahlenmäßig umfangreiche Tiergruppen, die in der modernen Nutztierhaltung im Vordergrund steht, aus Kostengründen nicht praktikabel ist. Auch hier stellt die Trinkwassermedikierung eine sinnvolle, nützliche Alternative dar. Es zeigt sich also, daß der Bereitstellung einer den praktischen Bedürfnissen angepaßten stabilen Trinkwasserlösung von Sulfonamid-Trimethoprim-Kombinationen für die Bekämpfung von Infektionserkrankungen bei Tieren eine wichtige Bedeutung zukommt. Darüber hinaus stellt die Trinkwassermedikierung für einige Nutztierarten wie beispielsweise Geflügel in der Praxis die einzige Möglichkeit zur Therapie größerer Tiergruppen dar.

Die Bereitstellung von therapeutisch brauchbaren Trinkwasser-Lösungen eines Sulfonamids in Kombination mit Trimethoprim bereitet indessen beträchtliche Schwierigkeiten, wobei sowohl Mängel in der Beständigkeit der Konzentrate als auch die Instabilität vor allem der verteilungskonform verdünnten Wirkstofflösungen eine Hauptrolle spielen. Das gilt um so mehr als heutzutage in immer stärkerem Maße automatische Tränkeeinrichtungen Eingang in die Praxis finden. Ein reibungsloses Funktionieren derartiger Vorrichtungen hängt jedoch u. a. auch von der einwandfreien Beschaffenheit der zur Verteilung gelangenden Lösungen ab. So führt das Auftreten von Ausfällungen wie festen Niederschlägen in den für die Herstellung der Endverdünnung vorgesehenen Lösungen zwangläufig zu Schwankungen in der Dosierung, wodurch eine zuverlässige, kontrollierte Therapie nicht mehr gewährleistet ist. Ferner kann das Auftreten von festen Bestandteilen in den Zwischenverdünnungen der die Wirkstoffe enthaltenden Trinkwasserlösungen Störungen in den Verteilungsapparaturen zur Folge haben, was mit Verlusten an wertvollem Material und zusätzlichem Arbeitsaufwand verbunden ist.

Da nun weder Trimethoprim noch die Sulfonamide eine ausreichende Wasserlöslichkeit besitzen, werden im allgemeinen die gut wasserlöslichen Salze dieser Verbindungen verwendet. Hierbei ergibt sich jedoch das Problem, daß bei Kombination der gebildeten Salzlösungen durch Annäherung des pH-Wertes an den Neutralpunkt eine Ausfällung der Wirksubstanzen stattfindet. Zur Behebung dieser Schwierigkeiten ist bereits mehrfach vorgeschlagen worden, Wirkstoffkombinationen bestehend aus Trimethoprim und jeweils verschiedenen Sulfonamiden unter Verwendung von organischen Lösungsmitteln bzw. Lösungsvermittlern in eine für therapeutische Zwecke geeignete Form zu bringen. So wird in der Deutschen Offenlegungsschrift Nr. 2 445 401 die Herstellung von für Injektionszwecke bestimmten wasserfreien Wirkstoffkombinationslösungen mit einem Gemisch von Sulfadimiden und Sulfathiazol als Sulfonamidkomponente mit einer Konzentration von 24—48% beschrieben.

Die Deutsche Offenlegungsschrift Nr. 2 311 214 führt neben der Bildung von festen Sulfonamid-Trimethoprim-Kombinationen als Futterzusatz die Herstellung einer wasserarmen Injektionslösung mit einem Gesamtwirkstoffgehalt von ca. 25% an, wobei N-(4,5-Dimethyl-3-isoxazolyl)-sulfanilamid als Sulfonamid fungiert. Erwähnt werden dabei auch wäßrige Formulierungen, bei denen der Gehalt der genannten Wirkstoffkombination für orale Anwendung durch Verdünnen mit Wasser auf 5% und darunter gesenkt werden kann.

Die britische Patentschrift Nr. 1 176 395 berichtet über injizierbare Formulierungen von Trimethoprim mit einer Reihe von Sulfonamiden wie 5,6-Dimethoxy-4-sulfanilamidpyrimidin, 4,6-Dimethyl-2-sulfanilamidpyrimidin, 2,4-Dimethoxy-6-sulfanilamidpyrimidin oder 3,4-Dimethyl-5-sulfonamidisoxazol mit Wirkstoffkonzentrationen von 12—14%. Für die Herstellung dieser Formulierungen müssen zuerst die Wirkstoffkomponenten voneinander getrennt in Lösung gebracht werden, wobei die Sulfonamide entweder zusammen mit Basen oder in einem Fall als Na-Salz in Wasser und das Trimethoprim in organischen Lösungsmitteln oder Lösungsvermittlern gelöst werden, um dann miteinander vermischt zu werden.

Dagegen wird für die Herstellung der in der Deutschen Offenlegungsschrift Nr. 2 400 218 beschriebenen flüssigen Formulierungen von Trimethoprim mit Sulfamethazol oder Sulfacetamid eine andere Verfahrensweise angegeben. So wird zuerst das Trimethoprim unter Zusatz von Säure in Wasser gelöst und dann das Sulfonamid zusammen mit organischen Lösungsmitteln bzw. Lösungsvermittlern hinzugegeben. Die so hergestellten Wirkstoffkombinationen werden gemäß der obengenannten Patentschrift zur Injektion und für orale Applikation vorgeschlagen. Ihre Wirkstoffkonzentration beträgt ca. 8—10% und z. T. auch weniger als 1%.

Eine Überprüfung der in der Deutschen Offenlegungsschrift Nr. 2 311 214 und der britischen Patentschrift Nr. 1 176 395 vorgeschlagenen Formulierungen konnte jedoch hinsichtlich ihrer Träger- und Verteilungsstoffe bei Anwendung auf die Wirkstoffkombinationen der vorliegenden Erfindung nicht befriedigen, so daß in der Praxis brauchbare stabile und klare Lösungen mit diesen Wirkstoffen nicht herstellbar waren.

Die Vorbeschreibungen betreffen also vor allem Sulfonamid-Trimethoprim-Formulierungen höherer Konzentration mit und ohne Wasserzusatz, die in erster Linie für die Injektionsanwendung vorgesehen sind, jedoch mitunter auch für die Trinkwasserapplikation empfohlen werden. In den meisten Fällen ist dafür die Verdünnung mit Wasser notwendig, um den Wirkstoffgehalt auf die für diese Verwendung erforderliche geringere Konzentration zu bringen, wobei es im Konzentrationsbereich der für automatische Tränkevorrichtungen notwendigen Zwischenverdünnungen mehr oder weniger schnell zu Ausfällungen kommt, was die Unbrauchbarkeit dieser Lösungen zur Folge hat. Bei den bereits für die Trinkwasserapplikation vorgeschlagenen Formulierungen besteht der Nachteil, daß die Zwischenverdünnungen nur sehr kurze Zeit klar bleiben und keine Aufbewahrung gestatten, was je nach Applikationsfolge somit die mehr oder weniger häufige Herstellung von frischen, zum möglichst raschen Verbrauch bestimmten Wirkstofflösungen in Wasser unabdingbar macht.

Das führt in der Praxis vor allem bei gleichzeitiger Behandlung größerer Gruppen von erkrankten Tieren sowie bei prophylaktischer Medikierung, die im allgemeinen auf breiter Basis durchgeführt wird, zu einem sehr nachteiligen arbeits- und kostenträchtigen Aufwand.

Außerdem wird in Publikationen darauf hingewiesen, daß einige der in den vorgenannten Patentschriften bzw. Offenlegungsschriften als funktionelle Hilfstoffe vorgeschlagenen Lösungsmittel bzw. Lösungsvermittler wie beispielsweise N,N-Dimethylacetamid, Propylenglykol, Dimethylformamid, Glycerinformal und Polyäthylenglykole aus pharmakologisch-toxischer-Sicht als nicht ganz unbedenklich angesehen werden können (siehe dazu DE-A-2 631 779; H. P. Fiedler, Lexikon der Hilfsstoffe für Pharmazie, Kosmetik, 1971; Prax. Pneumolog. 28 (1971) 491).

Ausgehend von den bereits erwähnten Vorteilen, die in der Verabreichung von Medikamenten von Sulfonamid-Trimethoprim-Kombinationen zur prophylaktischen und therapeutischen Behandlung hinsichtlich der Trinkwasserapplikation gegenüber der Verabreichung in Form von Zusätzen zu Trockenfutter oder Injektionen a priorie bestehen, ergibt sich das dringende Bedürfnis wäßrige Lösungen der genannten Wirkstoffkombinationen herzustellen, die bezüglich Klarheit und Löslichkeit in einem breiten Konzentrationsbereich und über einen längeren Zeitraum stabil sind, um den Erfordernissen der Praxis besser als bisher zu genügen.

Zur Lösung dieser Aufgabe werden deshalb mit Wasser verdünnbare flüssige Formulierungen folgender Zusammensetzung vorgeschlagen:

2,6-Diamino-5-(3',4',5'-trimethoxybenzyl)-pyrimidin (Trimethoprim) und ein Sulfonamid der Formel I

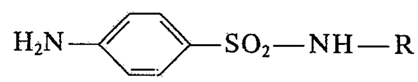

(I)

in welcher

R 6-Chlor-2-pyrazinyl oder 6-Chlor-3-pyridazinyl bedeutet, oder ein Salz davon

im Mengenverhältnis 1 : 1 bis 1 : 20, vorzugsweise 1 : 4 bis 1 : 5, mit einem Gesamtanteil von 15 bis 25 Gew.-% gelöst in einem physiologisch verträglichen, mit Wasser mischbaren Lösungsmittel, einem Lösungsvermittler und einem Tensid und für den Fall, daß das Sulfonamid nicht als Salz eingesetzt wird, einer Base, dadurch gekennzeichnet, daß als Lösungsmittel N-Methylpyrrolidon als Tensid Bis(2-äthylhexyl)-Na-sulfosucccinat oder das Polyoxyäthylensorbitan-monolaurat Polysorbatum 20, als Lösungsvermittler Hydroxyäthyltheophyllin oder Polyvinylpyrrolidon K 25 und als Base Äthanolamin verwendet werden. Bezüglich der eingesetzten Mengen kommen vorzugsweise 70—90 Gew.-% Lösungsmittel, 0,1—1,0 Gew.-% Tensid sowie 1—10 Gew.-% Lösungsvermittler in Betracht. Bei den zur Anwendung gelangenden Lösungsvermittlern sind folgende Mengen als besonders bevorzugt anzusehen für Hydroxyäthyltheophyllin 1—5 Gew.-% und Polyvinylpyrrolidon K 25 2—4 Gew.-%.

Unter den beiden vorstehend genannten Wirkstoffkombinationen ist diejenige neu, die neben Trimethoprim als Sulfonamid N-[6-Chlor-pyrazinyl-(2)]-sulfanilamid(Sulfachlorpyrazin) enthält, während eine Kombination enthaltend außer Trimethorpim als Sulfonamid N-[6-Chlor-pyridazinyl-(3)]-sulfanilamid(Sulfachlorpyridazin) bezüglich ihrer Wirkstoffe bereits vorbeschrieben ist (vgl. Magyar Allator vosok

Lapja 12 (1975) 833—836). In der erfindungsgemäß vorgeschlagenen speziellen Komposition zusammen mit den verwendeten funktionellen Hilfsstoffen ist sie jedoch neu.

Mit den vorliegenden erfindungsgemäßen Formulierungen ist es gelungen, konzentrierte wasserfreie Lösungen herzustellen, die nicht nur über längere Zeit klar bleiben und unter normalen Bedingungen unbeschadet lagerungsfähig sind, sondern auch noch nach Verdünnung mit Wasser über einen breiten Konzentrationsbereich hinweg überraschenderweise ihre Stabilitätseigenschaften bewahren. So sind einerseits die konzentrierten Formen den lagerungs- und transportbedingten Belastungen gegenüber stabil, während andererseits die verdünnten Lösungen als Zwischenverdünnungen über eine dem Bedarf angepaßte Zeitspanne von mehreren Tagen am Einsatzort stabil aufbewahrt werden können, um dann ohne besonderen Aufwand in die gewünschte, den therapeutischen Erfordernissen entsprechende Endverdünnung überführt zu werden. Der dadurch ermöglichte einfache und ohne Verluste an wertvollen Wirkstoffen arbeitende Dosierungs- und Applikationsmodus dient insbesondere einer Verbesserung der therapeutischen Effizienz durch präzisere Wirkstoffdosierung sowie der Verminderung des Aufwands an Material und Arbeit.

Das auf den erfindungsgemäßen Formulierungen basierende vorteilhafte Verdünnungsschema wird durch folgende Verhältnisse charakterisiert: In den Formulierungen beträgt der Anteil an Gesamtwirkstoff im allgemeinen 15—25 Gew.-%. Daraus werden mengenmäßig je nach Anwendungsbedarf klare Zwischenverdünnungen hergestellt, in denen durch Zugabe von Trinkwasser im Verhältnis 5 : 1 bis 50 : 1 die Wirkstoffkonzentration entsprechend herabgesetzt ist, ohne daß die Stabilität der Zwischenverdünnungen darunter leidet. Die über einen Zeitraum von mehreren Tagen stabilen Wirkstoffzwischenverdünnungen werden in den Verteilungsvorrichtungen (Dispensem) am Einsatzort unter normalen Temperaturbedingungen aufbewahrt. Die für die Behandlung der Tiere erforderlichen Mengen an Wirkstofflösung werden dann diesen Dispensern automatisch entnommen, wobei durch entsprechende Dosierungsvorrichtungen unter Zugabe von Trinkwasser die für die Therapie jeweils benötigte Endkonzentration präzise eingestellt wird. Bei dieser Endverdünnung liegt der Wirkstoffanteil im allgemeinen unter 1 Gew.-%, teilweise sogar unter 0,1 Gew.-% bis zu 0,01 Gew.-%. Die Stabilitätseigenschaften der erfindungsgemäßen Formulierungen, die auch bei starken Verdünnungen mit Wasser vorliegen, gestatten durch Anwendung des beschriebenen Verdünnungsprinzips eine exakte Festlegung der zur Applikation gelangenden Wirkstoffdosis, womit eine genaue Kontrolle und Steuerung der jeweiligen Therapie gegeben ist.

Gegen die zur Herstellung der erfindungsgemäßen Formulierungen verwendeten funktionellen Hilfsstoffe wie Lösungsmittel und Lösungsvermittler sind hinsichtlich ihrer toxikologischen Unbedenklichkeit bei oraler Verabreichung in der erfindungsgemäß beschriebenen Weise in der Fachwelt bisher keine Einwände bekannt geworden.

Die in den erfindungsgemäßen Formulierungen enthaltenen Wirkstoffkombinationen besitzen ein breites antibakterielles und antiprotozoäres Wirkungsspektrum, unter anderem vor allem gegen Escherichia coli, Kokzidien, Salmonellen, Staphylokokken und Streptokokken und sind deshalb zur prophylaktischen und therapeutischen Behandlung von Infektionskrankheiten bei Haus- und Nutztieren wie Geflügel, Schweinen, Kälbern, Rindern, Schafen und Ziegen besonders geeignet. Dabei gewinnen die erfindungsgemäßen Formulierungen als Wirkstoffträger für die Trinkwassermedikation dank der Hervorbringung von hervorragenden Löslichkeitseigenschaften gerade für die Behandlung von größeren Tierbeständen, wie sie heutzutage besonders in der landwirtschaftlichen Intensivproduktion üblich sind, eine wichtige Bedeutung.

Die in den erfindungsgemäßen Formulierungen verwendeten Wirkstoffe sind bekannt. So ist das 2-Diamino-5-(3',4',5'-trimethoxybenzyl)-pyrimidin (Trimethoprim) in Brit. Pharmakopoeia 1973, 484 und das N-[6-Chlorpyridazinyl-(3)]-sulfanilamid (Sulfachlorpyridazin) in Handbook of Veterinary Drugs, Ed. Springer New York 1974, 564 sowie in Ullmanns Enzyklopädie d. Techn. Chemie, Verlag Chemie Weinheim, 3. Aufl. Bd. 15 und das N-[6-Chlorpyrazinyl-(2)]-sulfanilamid (Sulfachlorpyrazin) in Handbook of Veterinary Drugs, Ed. Springs New York 1974, 564 beschrieben.

Ferner sind die als funktionelle Hilfsstoffe verwendeten Lösungsmittel, Lösungsvermittler und Tenside der Fachwelt als solche bekannt.

Die erfindungsgemäßen Wirkstofformulierungen werden nach folgendem Verfahren hergestellt, indem man die Wirkstoffe zusammen mit einem Lösungsvermittler, einem Tensid in einem geeigneten abdunkelbaren Gefäß, möglichst unter Ausschluß von Licht unter ständigem Rühren im größten Teil des vorgesehenen Lösungsmittels bei 10 bis 70° C, vorzugsweise 10° bis 50° C, löst, anschließend mit dem Rest des Lösungsmittels auffüllt und falls nötig unter Verwendung von Filtrierhilfsmitteln, beispielsweise Kieselgur, filtriert. Zur Beschleunigung der Filtration kann die Temperatur gegenüber dem Lösungsvorgang leicht erhöht werden.

Die folgenden Beispiele, die die erfindungsgemäßen Wirkstofformulierungen veranschaulichen, wurden bei Raumtemperatur durchgeführt, wobei unter Raumtemperatur gemäß Ph. Eur. Bd I der Temperaturbereich von 15° bis 25° C zu verstehen ist.

### Beispiel 1

In ein geeignetes, abdunkelbares Gefäß werden 15,48 g Sulfachlorpyridazin, 3,33 g Trimethoprim, 3,0 g Hydroxyäthyltheophyllin, 0,5 g Natrium-dioctylsufosuccinat sowie 4,0 g Äthanolamin eingewogen und unter wiederholtem Umschütteln in ca. 80,0 g N-Methylpyrrolidon gelöst. Die Lösung wird mit weiteren ca. 5,0 g N-Methylpyrrolidon auf 100 ml aufgefüllt und nötigenfalls unter Zusatz eines Filterhilfsmittels filtriert.

Beispiel 2

In ein geeignetes, abdunkelbares Gefäß werden 14,72 g Sulfachlorpyrazin, 3,33 g Trimethoprim, 3,0 g Polyvinylpyrrolidon K 25, 0,5 g Natrium-diotylsulfosuccinat sowie 4,0 g Äthanolamin eingewogen und unter wiederholtem Umschütteln in ca. 80,0 g N-Methylpyrrolidon gelöst. Die Lösung wird mit weiteren ca. 5,0 g N-Methylpyrrolidon auf 100 ml aufgefüllt und nötigenfalls unter Zusatz eines Filterhilfsmittels filtriert.

Beispiel 3

15,48 g Sulfachlorpyridazin, 3,33 g Trimethoprim, 3,0 g Polyvinylpyrrolidon K 25 und 0,5 Natrium-dioctylsulfosuccinat werden in 4,0 g Äthanolamin und ca. 80 g N-Methylpyrrolidon in einem geeigneten, abdunkelbaren Gefäß unter wiederholtem Umschütteln gelöst, mit weiteren ca. 4 g N-Methylpyrrolidon auf 100 ml aufgefüllt und nötigenfalls unter Verwendung eines Filterhilfsmittels filtriert.

Beispiel 4

15,48 g Sulfachlorpyridazin, 3,33 g Trimethoprim, 3,0 g Hydroxyäthyltheophyllin und 0,5 g des Polyoxyäthylensorbitanmonolaurats Polysorbatum 20 werden in einem geeigneten, abdunkelbaren Gefäß unter wiederholtem Umschütteln in ca. 80 g N-Methylpyrrolidon und 4,0 g Äthanolamin gelöst, mit weiteren ca. 4 g N-Methylpyrrolidon auf 100 ml aufgefüllt und, wenn nötig unter Verwendung eines Filterhilfsmittels, filtriert.

Beispiel 5

15,48 g Sulfachlorpyridazin, 3,33 g Trimethoprim, 2,0 g Hydroxyäthyltheophyllin und 0,5 g Natrium-dioctylsulfosuccinat werden in einem geeigneten, abdunkelbaren Gefäß unter wiederholtem Umschütteln in ca. 80 g N-Methylpyrrolidon und 4,0 g Äthanolamin gelöst, mit weiteren ca. 5 g N-Methylpyrrolidon auf 100 ml aufgefüllt und, wenn nötig unter Verwendung eines Filterhilfsmittels, filtriert.

**Patentansprüche**

1. Stabile flüssige Formulierung enthaltend 2,6-Diamino-5-(3′,4′,5′-trimethoxybenzyl)-pyrimidin (Trimethoprim) und ein Sulfonamid der Formel I

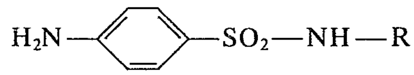 (I)

in welcher

R 6-Chlor-2-pyrazinyl oder 6-Chlor-3-pyridazinyl bedeutet, oder ein Salz davon

im Mengenverhältnis 1 : 1 bis 1 : 20 vorzugsweise 1 : 4 bis 1 : 5 mit einem Gesamtanteil von 15—25 Gew.-%, ein physiologisch verträgliches mit Wasser mischbares Lösungsmittel, einen Lösungsvermittler und ein Tensid und für den Fall, daß das Sulfonamid nicht als Salz eingesetzt wird, einer Base, dadurch gekennzeichnet, daß als Lösungsmittel N-Methylpyrrolidon, als Tensid Bis-(2-äthylhexyl)-Na-sulfosuccinat oder das Polyoxyäthylensorbitan-monolaurat Polysorbatum 20, als Lösungsvermittler Hydroxyäthyltheophyllin oder Polyvinylpyrrolidon K 25 und als Base Äthanolamin verwendet werden.

2. Stabile flüssige Formulierung gemäß Anspruch 1, dadurch gekennzeichnet, daß die verwendete Menge des Lösungsmittels 70—90 Gew.-% des Tensids 0,1—1,0 Gew.-% und des Lösungsvermittlers 1—10 Gew.-% beträgt.

3. Stabile flüssige Formulierung gemäß Anspruch 1 und 2, dadurch gekennzeichnet, daß als Lösungsvermittler 1—5 Gew.-% Hydroxyäthyltheophyllin verwendet wird.

4. Stabile flüssige Formulierung gemäß Anspruch 1 und 2, dadurch gekennzeichnet, daß als Lösungsvermittler 2—4 Gew.-% Polyvinylpyrrolidon K 25 verwendet wird.

5. Verfahren zur Herstellung einer Wirkstoffkombination gemäß den Ansprüchen 1—4, dadurch gekennzeichnet, daß man die Wirkstoffe zusammen mit einem Lösungsvermittler Hydroxyäthyltheophyllin oder Polyvinylpyrrolidon K 25, einem Tensid Bis-(2-äthylhexyl)-Na-sulfosuccinat oder das Polyoxyäthylensorbitanmonolaurat Polysorbatum 20 im größten Teil des Lösungsmittels N-Methylpyrrolidon bei 10° bis 70° C, vorzugsweise 10° bis 50° C, löst und anschließend die gebildete Lösung mit dem Rest des Lösungsmittels auffüllt und gegebenenfalls unter Anwendung von Filtrierhilfsmitteln und Temperaturerhöhung filtriert.

6. Verwendung der Formulierung gemäß der Ansprüche 1—4, dadurch gekennzeichnet, daß diese in durch Trinkwasser zwischenverdünnter Form mit automatischen Dosiergeräten den zur Verabreichung an Tiere vorgesehenen üblichen Tränken laufend beigemischt werden.

**Claims**

1. A stable liquid formulation containing 2,6-diamino-5-(3′,4′,5′-trimethoxybenzyl)-pyrimidine (trimethoprim) and a sulfonamide of the formula I

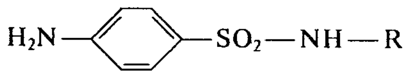 (I)

in which

R is 6-chloro-2-pyrazinyl or 6-chloro-3-pyridazinyl, or a salt thereof,

in the quantity ratio of 1 : 1 to 1 : 20, preferably 1 : 4 to 1 : 5, with a total proportion of 15 to 25 per cent by weight being dissolved in a physiologically compatible, watermiscible solvent, a solubility-promoting agent and a surfactant, and a base in the case where the sulfonamide is not used as a salt, in which composition the solvent used is N-methylpyrrolidone, the surfactant used is bis-(2-ethylhexyl)-Na-sulfosuccinate or polyoxyethylene-sorbitan monolaurate (polysorbaum 20), the solubility-promoting agent used is hydroxyethyltheophylline or polyvinylpyrrolidone K 25, and the base used is ethanolamine.

2. A stable liquid formulation according to Claim 1, wherein the amount of solvent used is 70—90 per cent by weight, the amount of surfactant used is 0.1—1.0 per cent by weight, and the amount of solubility-promoting agent used is 1—10 per cent by weight.

3. A stable liquid formulation according to Claims 1 and 2, wherein there is used as the solubility-promoting agent 1—5 per cent by weight of hydroxyethyltheophyline.

4. A stable liquid formulation according to Claims 1 and 2, wherein there is used as the solubility-promoting agent 2—4 per cent by weight of polyvinylpyrrolidone K 25.

5. A process for producing an active-substance combination according to Claims 1—4, wherein the active substances, together with a solubility-promoting agent, namely hydroxyethyltheophylline or polyvinylpyrrolidone K 25, and a surfactant, namely bis-(2-ethylhexyl)-Na-sulfosuccinate or polyoxyethylene sorbitan monolaurate (polysorbatum 20) are dissolved in the major part of the solvent N-methylpyrrolidone at 10° to 70° C, preferably at 10° to 50° C, the formed solution is subsequently made up to the required amount with the remainder of the solvent, and is then filtered, if necessary using filtering auxiliaries and raising the temperature of the solution.

6. Use of the formulation according to Claims 1—4, which comprise continuously mixing this formulation, in the form it is in after it has been intermediately diluted with drinking water, by means of automatic dosing devices, with the customary drinkung water being supplied to the animals.

**Revendications**

1. Formulation liquide stable contenant de la 2,6-diamino-5-(3′,4′,5′-triméthoxybenzyl)-pyrimidine (Trimethoprim) et un sulfonamide de formule I

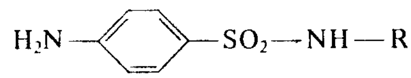 (I)

dans laquelle

R représente le groupe 6-chloro-2-pyrazinyle ou le groupe 6-chloro-3-pyridazinyle,

ou un sel d'un tel composé, dans des proportions relatives de 1 : 1 à 1 : 20, de préférence de 1 : 4 à 1 : 5, avec une teneur totale de 15 à 25% en poids, un solvant miscible à l'eau acceptable pour l'usage pharmaceutique, un agent solubilisant et un agent tensio-actif et, dans le cas où le sulfonamide n'est pas mis en œuvre à l'état de sel, une base, caractérisée en ce que l'on utilise en tant que solvant la N-méthylpyrrolidone, en tant qu'agent tensio-actif le sel de sodium du sulfosuccinate de bis-(2-éthylhexyle) ou le monolaurate de sorbitanne polyoxyéthyléné Polysorbatum 20, en tant qu'agent solubilisant l'hydroxyéthylthéophylline ou la polyvinylpyrrolidone K 25 et en tant que base l'éthanolamine.

2. Formulation liquide stable selon la revendication 1, caractérisée en ce que les quantités utilisées vont de 70 à 90% en poids pour le solvant, de 0,1 à 1,0% en poids pour l'agent tensio-actif et de 1 à 10% en poids pour l'agent solubilisant.

3. Formulation liquide stable selon les revendications 1 et 2, caractérisée en ce que l'on utilise en tant qu'agent solubilisant l'hydroxyéthylthéophylline en proportion de 1 à 5% en poids.

4. Formulation liquide stable selon les revendications 1 et 2, caractérisée en ce que l'on utilise en tant qu'agent solubilisant la polyvinylpyrrolidone K 25 en proportion de 2 à 4% en poids.

5. Procédé de préparation d'une combinaison de substances actives selon le revendications 1 à 4, caractérisé en ce que l'on dissout les substances actives avec un agent solubilisant, l'hydroxyéthylthéophylline ou la polyvinylpyrrolidone K 25, un agent tensio-actif, le sel de sodium du sulfosuccinate de bis-(2-éthylhexyle) ou le monolaurate de sorbitanne polyoxyéthyléné Polysorbatum 20, dans la plus grande partie du solvant, la N-méthylpyrrolidone, à une température de 10 à 70° C, de préférence de 10 à 50° C, on complète ensuite le volume de la solution formée par le reste du solvant et le cas échéant on filtre en utilisant des agents filtrants et en augmentant la température.

6. Utilisation de la formulation selon l'une des revendications 1 à 4, caractérisée en ce que celleci, à une dilution intermédiaire obtenue par de l'eau de boisson, est mélangée en continu au moyen de dispositifs doseurs automatiques aux boissons usuelles prévues pour l'administration aux animaux.